(19) Europäisches Patentamt

European Patent Office

Office européen des brevets

(11) Veröffentlichungsnummer: **0 475 354 A2**

# EUROPÄISCHE PATENTANMELDUNG

(21) Anmeldenummer: **91115282.5**

(22) Anmeldetag: **10.09.91**

(51) Int. Cl.5: **C12N 15/15**, C12N 15/16, C12N 15/54, C12P 21/00, C07K 13/00

(30) Priorität: **11.09.90 DE 4028800**

(43) Veröffentlichungstag der Anmeldung:
**18.03.92 Patentblatt 92/12**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**W-3550 Marburg 1(DE)**

(72) Erfinder: **Zettlmeissl, Gerd, Dr.**
**Elbingerstrasse 3**
**W-3552 Wetter(DE)**

Erfinder: **Grundmann, Ulrich, Dr.**
**Am Pfahltor 7**
**W-3551 Lahntal(DE)**
Erfinder: **Becker, Achim**
**Oberlandstrasse 4**
**W-3563 Dautphetal-Holzhausen(DE)**
Erfinder: **Hermentin, Peter, Dr.**
**Salzköppel 9**
**W-3550 Marburg(DE)**

(74) Vertreter: **Aulmich, Gerhard, Dr. et al**
**Hoechst AG Zentrale Patentabteilung**
**Postfach 80 03 20**
**W-6230 Frankurt am Main 80(DE)**

(54) **Gentechnische Sialylierung von Glykoproteinen.**

(57) Die Erfindung betrifft die gentechnische Sialylierung von Glykoproteinen sowie neue sialylierte Glykoproteine ("übersialylierte Glykoproteine"). Die gentechnische Sialylierung wird dadurch bewirkt, daß man eine für eine Sialyltransferase kodierende DNA-Sequenz zusammen mit einer für das zu sialylierende Glykoprotein kodierenden DNA-Sequenz in einer eukaryontischen Zelle zur Expression bringt und das gebildete sialylierte Glykoprotein isoliert.

Die vorliegende Erfindung betrifft die gentechnische Sialylierung von Glykoproteinen sowie neue sialylierte Glykoproteine ("übersialylierte Glykoproteine").

Eine Reihe von für therapeutische Zwecke einsetzbaren Glykoproteinen, wie z.B. Factor VIII.C,Factor IX, Erythropoietin, Antithrombin III und monoklonale Antikörper, können mit Hilfe bekannter Verfahren in Säugerzellen, die in der Lage sind, komplexe Kohlenhydratseitenketten aufzubauen, synthetisiert werden (Kaufman et al., J.Biol.Chem. Bd. 263 (1988), 6352-6362; Kaufman et al., J.Biol.Chem. Bd. 261 (1986), 9622-9628; Lin et al., Proc.Natl.Acad.Sci. USA, Bd. 82 (1985), 7580-7584; Zettlmeißl et al., Bio/Technology, Bd. 5, (1987) 720-725, Köhler und Milstein, Nature 256 (1975) 495-497). Bei der biochemischen Charakterisierung der Kohlenhydratseitenketten derart hergestellter rekombinanter Glykoproteine zeigte sich, daß in Abhängigkeit vom verwendeten Expressionssystem ein rekombinantes Glykoprotein im Vergleich zum natürlich vorkommenden Glykoprotein Unterschiede im Glykosylierungsmuster aufweist (Pfeiffer et al., Eur.J.Biochem., Bd. 186 (1989), 273-286. Zettlmeißl et al., J.Biol.Chem., Bd. 264 (1989), 21153-21159). So wird häufig in Chinese Hamster Ovary (CHO) Zellen bei der Synthese rekombinanter Glykoproteine terminale Sialinsäure ausschließlich in $\alpha$-2,3-Verknüpfung an die Kohlenhydratseitenketten von z.B. rekombinantem Erythropoietin (Takeuchi et al., J.Biol.Chem., Bd. 263 (1988), 3657-3663) oder rekombinantem Antithrombin III (Zettlmeißl et al., J.Biol.Chem., Bd. 264 (1989), 21153-21159) angefügt. Hingegen findet man bei natürlichen Formen vorwiegend $\alpha$-2,6-verknüpfte Sialinsäure.

Desweiteren konnte bei komplex glykosylierten Proteinen, die aus natürlichen Quellen (z.B. Humanurin oder Humanplasma) isoliert wurden (Franzen et al., J.Biol.Chem. Bd. 255, 5090-5093) bzw. durch Expression in Säugerzellen erhalten wurden (Sasaki et al., J.Biol.Chem., Bd. 262 (1987), 12059-12076) eine unvollständige Sialylierung der Kohlenhydratseitenketten beobachtet werden. Es ist bekannt, daß verschiedene Glykoproteine, wie z.B. Erythropoietin oder Antithrombin III, bei unvollständiger Sialylierung eine kürzere Halbwertzeit in vivo aufweisen. Ferner ist bekannt, daß sogenannte "Asialoglykoproteine", welche auf ihren Zuckerketten an Stelle von terminaler Sialinsäure terminale Galactose aufweisen, eine besonders kurze Halbwertszeit in vivo besitzen. Derartige, unvollständig sialylierte Glykostrukturen werden nämlich von Rezeptoren in der Leber erkannt und aus dem Blutstrom rascher entfernt.

Es ist daher für eine in vivo Anwendung der Glykoproteine sehr wünschenswert und vorteilhaft, höher bzw. vollständig sialylierte Glykoproteine zur Verfügung zu haben, weil diese eine höhere Halbwertszeit in vivo und somit eine bessere Wirksamkeit besitzen, als die nativen Glykoproteine.

Bekannte Verfahren zur enzymatischen Sialylierung gereinigter Glykoproteine in vitro durch lösliche oder immobilisierte hochreine Sialyltransferasen weisen durch die geringe Verfügbarkeit der entsprechenden Enzyme und durch die aufwendige Synthese des Substrates (CMP-Neuraminsäure) für Sialyltransferase technische und wirtschaftliche Grenzen auf.

Der vorliegenden Erfindung liegt die Aufgabe zugrunde, höher bzw. komplett sialylierte Glykoproteine für therapeutische Zwecke bereitzustellen, die nach gentechnologischen Methoden hergestellt werden sollen. Die Lösung dieser Aufgabe wird dadurch erreicht, daß man eine für eine Sialyltransferase kodierende DNA-Sequenz zusammen mit einer für das zu sialylierende Glykoprotein kodierenden DNA-Sequenz in einer eukaryotischen Zelle zur Expression bringt und das gebildete sialylierte Glykoprotein isoliert.

Die cDNA-Sequenzen für die $\beta$-Galaktosid $\alpha$-2,6-Sialyltransferase (Gal $\alpha$-2,6-ST) aus Rattenleber (Weinstein et al., J.Biol.Chem., Bd. 262 (1987), 17735-17743) und aus menschlicher Plazenta (Grundmann et al., Nucl.Acids Res., Bd. 18 (1990), 667) wurden isoliert und charakterisiert. Die Gal $\alpha$-2,6-ST aus Rattenleber konnte in CHO-Zellen in funktioneller Form exprimiert werden. Dies hatte zur Folge, daß die von den CHO-Zellen synthetisierten CHO-Zellproteine neben der für diese Zell-Linie üblichen $\alpha$-2,3-verknüpften Sialinsäure auch Sialinsäure in $\alpha$-2,6-Verknüpfung aufwiesen (Lee et al., J.Biol.Chem., Bd. 264 (1989), 13848-13855).

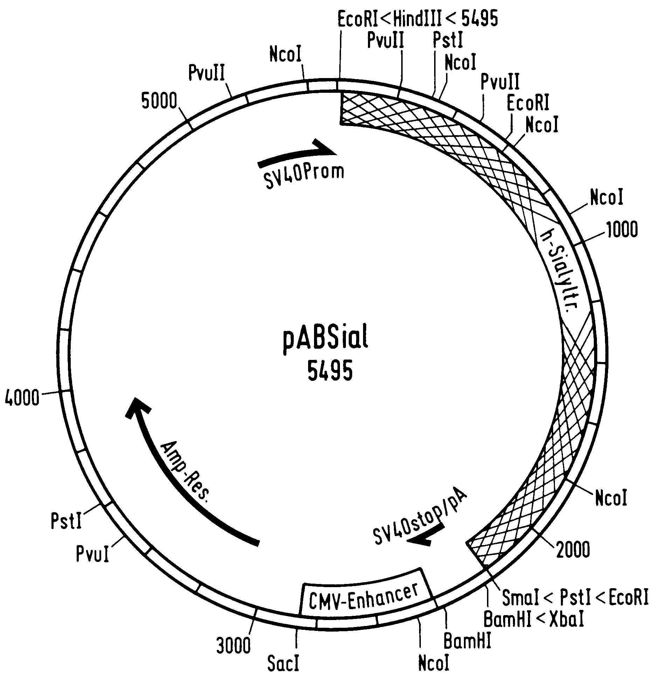

Erfindungsgemäß kann nun überraschenderweise ein heterologes Glykoprotein von therapeutischem Interesse durch Koexpression mit einer heterologen Sialyltransferase in einer eukaryotischen Zelle sowohl im Verknüpfungsmuster z.B. $\alpha$-2,3 vs. $\alpha$-2,6) der terminalen Sialinsäuren als auch vor allem in Bezug auf einen höheren Sialylierungsgrad, d.h. die möglichst vollständige Besetzung terminaler Galaktosereste mit Sialinsäure, verändert werden. Die gemäß dieser Erfindung erhaltenen hochsialylierten Glykoproteine zeichnen sich insbesondere durch langanhaltende Serumspiegel und damit durch eine verbesserte therapeutische Wirksamkeit aus. Ein weiterer Vorteil des Verfahrens ist dessen großtechnische Anwendbarkeit, was zu einheitlicheren Produkten ("homogene Sialylierung") führt. Zur Durchführung des Verfahrens der Erfindung wird die kodierende DNA-Sequenz für eine Sialyltransferase in bekannter Weise stromabwärts von einem starken eukaryotischen Promotor (z.B. SV40 early-Promotor, vgl. Fig. 2) kloniert und zusammen mit der Transkriptionseinheit für ein selektionierbares Markergen (z.B. Dihydrofolatreduktase, Aminoglykosid 3' Phosphotransferase, Puromycin N-Acetyltransferase, Wirth et al., Gene Bd. 73 (1988), 419-426; Glutaminsynthetase, Bebbington and Hentschel, DNA Cloning Vol. III (1987), Glover, ed., IRL press) in eine geeignete eukaryontische Zelle für die Herstellung des zu sialylierenden Glykoproteins eingeschleust und in dieser Produktionszelle zur Expression gebracht. Selektionierte eukaryotische Zellen, die die Sialyltransferase zusammen mit dem zu sialylierenden Glykoprotein exprimieren, können zur großtechnischen Synthese des Glykoproteins in hochsialylierter Form eingesetzt werden. Besonders bevorzugt als Markergene sind solche, die eine Koamplifikation der für Sialyltransferase kodierenden DNA-Sequenz erlauben, wie z.B. die cDNAs für Dihydrofolatreduktase oder Glutaminsynthetase.

Typische Beispiele für DNA-Sequenzen, die für Sialyltransferasen kodieren, sind die Sequenzen für Galaktosid $\alpha$-2,3-Sialyltransferase, Galaktosid $\alpha$-2,6-Sialyltransferase und N-Acetylgalactosamid $\alpha$-2,6-Sialyltransferase. Besonders bevorzugt ist die kodierende DNA-Sequenz für Galaktosid $\alpha$-2,6-Sialyltransferase (Weinstein et al., a.a.O.; Grundmann et al., a.a.O.).

Typische und bevorzugte Beispiele für das zu sialylierende rekombinante Glykoprotein sind: menschliches Antithrombin III (Bock et al., Nucl.Acids Res., Bd. 10 (1982), 8113-8125), menschliches Erythropoietin (Jacobs et al., Nature, Bd. 313 (1985), 806-809), menschlicher Faktor VII (Hagen et al., Proc.Natl.Acad.Sci. USA, Bd. 83 (1986), 2412-2416), menschlicher Faktor VIII.C (Toole et al., Nature, Bd. 312 (1984), 342-347), menschlicher Faktor IX (Choo et al., Nucl. Acids Res., Bd. 15 (1987), 871-884), glykosylierte menschliche Rezeptormoleküle, wie z.B. Gewebsfaktor (Fisher et al., Thromb.Res., Bd. 48 (1987), 89-99), Interleukin-1-Rezeptor (Sims et al., Proc.Natl.Acad.Sci. USA, Bd. 86 (1989), 8946-8950), Interleukin-4-Rezeptor (EP-A-0367 566), Interleukin-7-Rezeptor (Goodwin et al., Cell, Bd. 60 (1990), 941-951), Tumor-Nekrosis-Faktor-Rezeptor (Schall et al., Cell, Bd. 61 (1990), 361-370), CD4 (Maddon et al., Cell, Bd. 42 (1985), 93-104).

Das beanspruchte Verfahren kann in verschiedenen Varianten durchgeführt werden: (1) Zuerst wird die für Sialyltransferase kodierende DNA-Sequenz zusammen mit einem Markergen und dann die für das zu sialylierende Glykoprotein kodierende DNA-Sequenz zusammen mit einem weiteren Markergen in die eukaryontische Zelle eingebracht. (2) Zuerst wird die für das zu sialylierende Glykoprotein DNA-Sequenz zusammen mit einem Markergen und dann die für die Sialyltransferase kodierende DNA-Sequenz mit einem weiteren Markgergen in die eukaryontische Zelle eingebracht. (3) Die für die Sialyltransferase kodierende DNA-Sequenz und die für das zu sialylierende Glykoprotein kodierende DNA-Sequenz werden gleichzeitig zusammen mit einem Markergen in die eukaryotische Zelle eingebracht.

Typische Beispiele für eukaryontische Zellen sind Hefen, Pilze, Pflanzenzellen oder tierische Zellen. Bevorzugt für das Verfahren, das Gegenstand der vorliegenden Erfindung ist sind permanente Säugerzellen. Besonders bevorzugt sind Säugerzellen wie BHK, CHO, C127 die kommerziell erhältlich sind.

In einer weiteren Ausführungsform der Erfindung kann die für Sialyltransferase kodierende DNA-Sequenz mit einem Markergen in eine Zellinie eingebracht werden, die natürlicherweise Glykoproteine bilden. Bevorzugt sind hierbei Hybridomazellen, die monoklonale Antikörper bilden (Köhler und Milstein, a.a.O.) bzw. Zellen, die zur Vermehrung von Viren eingesetzt werden.

Die erfindungsgemäß erhaltenen sialylierten Proteine können nach Standardmethoden gereinigt und auf Sialylsäuregehalt analysiert werden (Hermentin und Seidat, GBF Workshop "Protein Glycosylation: Cellular, Biotechnological and Analytical Aspects", June 28-30, 1990, Braunschweig, Abstracts of Papers, p.49).

In der Figur und den Tabellen wird die Erfindung weiter erläutert:
Tabelle 1 zeigt die vollständige cDNA Sequenz des für menschliche Galaktosid $\alpha$-2,6-Sialyltransferase kodierenden Klons 14 mit seinen randständigen EcoRI-Linker-Anteilen. Die Figur zeigt das Expressionsplasmid für menschliche Galaktosid $\alpha$-2,6-Sialyltransferase pAB Sial.

In Tabelle 2 sind die Ergebnisse zur Sialinsäureverknüpfung von menschlichem Antithrombin III gereinigt aus Zellkulturüberständen von BHK-Zellen vor und nach Transfektion mit dem Plasmid pAB Sial zusammengefaßt.

Die Erfindung wird durch die Beispiele erläutert. Diese Beispiele sind nicht beschränkend aufzufassen. Weitere Angaben zu den hier angewendeten molekularbiologischen Verfahren sind zu finden in Sambrock et al., Molecular Cloning, 2. Auflage, 1989, Cold Spring Harbor Laboratory, Cold Spring Harbor, N.Y., U.S.A.

Beispiel 1: Klonierung der cDNA für humane $\beta$-Galaktosid 2,6-Sialyltransferase (Gal $\alpha$-2,6-ST)

Eine in der EP-A-0236 978 beschriebene cDNA-Bank aus menschlicher Placenta im Phagenvektor lambda gt10 wurde durch Hybridisierung mit synthetischen Oligonukleotid-Sonden auf Klone abgesucht, die eine für Gal $\alpha$-2,6-ST codierende cDNA tragen. Die beiden hierzu verwendeten Oligonukleotid-Sonden wurden aus der cDNA-Sequenz von Gal $\alpha$-2,6 ST der Ratte (Weinstein et al., a.a.O.) unter Berücksichtigung des Kodon-Gebrauchs in menschlichen proteinkodierenden Sequenzen (Lathe, J.Mol.Biol., Bd 183 (1985), 1-12) abgeleitet und hatten die folgenden Sequenzen:

3

```
A) 5'CTC CTG GCT CTT GGG CAT CTG GAA CTC CTT GGC CTG CAG
      GGT CAG GGC CTC ATA GTC 3' (57mer)


B) 5'ATG ATG ACC CTG TGT GAC CAG GTG GAC ATC TAT GAG TTC
      CTG CCA TCC AAG 3' (51mer)
```

Beide Oligonukleotidsequenzen wurden mit T4 Polynukleotidkinase unter Verwendung von (gamma-$^{32}$P) ATP am 5'-Ende markiert (äquimolare Mengen DNA und (gamma-$^{32}$P) ATP: 3000Ci/mmol, 10$\mu$Ci/$\mu$l, eingesetzt wurden 5$\mu$l/50$\mu$l pro Reaktionsansatz). Die Sonden hatten eine spezifische Aktivität von 2 x 10$^6$ Bq/$\mu$g bzw. 4 x 10$^4$ Bq/pMol.

Zum Absuchen der Placenta-cDNA Bank mit den vorstehend beschriebenen Oligonukleotidsonden wurden 3 x 10$^4$ pfu (plaque forming units) mit Zellen des E.coli K 12-Stammes C 600 hfl in Weich-Agar auf 13,5 cm Petrischalen ausplattiert und 6 h bei 37°C inkubiert. Nach diesem Zeitraum war noch keine vollständige Lyse eingetreten. Die Platten wurden über Nacht im Kühlschrank inkubiert und die Phagen auf Nitrocellulosefilter (Schleicher & Schüll, BA 85, Ref.-Nr. 401124) übertragen (Duplikate). Nitrocellulosefilter und Petrischalen wurden mit einer Injektionskanüle markiert, um eine spätere Zuordnung zu ermöglichen. Die Petrischalen wurden während der Weiterbehandlung der Nitrocellulosefilter im Kühlraum gelagert. Die auf den Nitrocellulosefiltern befindliche DNA wurde denaturiert, indem die Filter 5 min. auf ein in 1,5 M NaCl, 0,5 M NaOH-getränktes Filterpapier (Whatman-3M) gelegt wurden. Anschließend wurden die Filter auf die gleiche Art mit 1,5 M NaCl, 0,5 M Tris (pH 8,0) renaturiert und mit 2 x SSPE (0,36 M NaCl, 16 mM NaOH, 20 mM NaH$_2$PO$_4$, 2 mM EDTA) gewaschen. Die Filter wurden dann 2 h bei 80°C unter vermindertem Druck gebacken. Die Filter wurden 4 h bei 65°C prähybridisiert (Prähybridisierungslösung: 0,6 M NaCl, 0,06 M Tris (pH 8,3), 6 mM EDTA, 0,2 % nichtionisches synthetisches Saccharose-Polymer (Ficoll$^R$), 0,2 % Polyvinylpyrrolidon 40, 0,2 % BSA, 0,1 SDS, 50 $\mu$g/ml denaturierte Heringssperma-DNA). Schließlich wurden die Filter über Nacht unter Zusatz von 100 000 - 200 000 bq des markierten Oligonukleotids/ml Hybridisierungslösung (wie Prähybridisierungslösung, jedoch ohne Heringssperma-DNA) in Bechergläsern bzw. in zugeschweißten Polyethylenfolien unter leichtem Schütteln inkubiert. Die Hybridisierungstemperatur betrug 65°C. Die Nitrocellulosefilter wurden mit 6 x SSC, 0,05 M Natriumpyrophosphat eine Stunde bei Raumtemperatur und eine weitere Stunde bei Hybridisierungstemperatur gewaschen. Die Filter wurden getrocknet und über Nacht autoradiographiert. Signale auf dem Röntgenfilm, die bei beiden Duplikaten in identischer Position auftraten, wurden der Petrischale zugeordnet und die Region (ca. 30 Plaques) mit dem breiten Ende einer Pasteurpipette ausgestanzt und die Phagen in 1 ml SM-Puffer resuspendiert. Phagengemische, die Phagen mit der Sonde hybridisierenden cDNAs enthielten wurden über drei Runden vereinzelt, bis ein einzelner positiver Klon identifiziert werden konnte. Insgesamt wurden 10$^6$ pfu der Placenta-cDNA Bank abgesucht und zwei Phagen, die positive Signale ergaben, wurden näher analysiert (Klone Nr. 14 und 15). Die beiden Phagenklone 14 und 15 wurden vermehrt und ihre DNAs extrahiert. Die DNAs wurden partiell mit EcoRI gespalten, isoliert und in die EcoRI-Restriktionsschnittstelle des Vectors Bluescript M13 (Stratagene, San Diego, CA, USA) für Restriktions- und Sequenzanalysen ligiert. Die entstehenden rekombinanten Plasmide wurden je nach Orientierung des EcoRI-Fragmentes pSial14 (L oder R) und pSial15 (L oder R) genannt. Sie enthielten alle die gesamte kodierende Sequenz. Tab. 1 zeigt die vollständige cDNA-Sequenz von Klon 14 (2188 bp) mit seinen randständigen EcoRI-Linker-Anteilen (5'AG 'AATTCT). Die Sequenz zeigt einen offenen Leseraster und kodiert für ein Protein von 406 Aminosäuren.

Beispiel 2: Koexpression von menschlicher Gal $\alpha$-2,6-ST in einer Baby Hamster Kidney (BHK)-21 Zelle, die menschliches Antithrombin III (AT III) exprimiert

a) Herstellung einer BHK21-Zell-Linie, die menschliches AT III exprimiert

Die Herstellung des für die weiteren Experimente verwendeten BHK-Mischklons 3MK1, der menschliches AT III in das Kulturmedium sezerniert, ist in Beispiel 1b der EP-A-0330 977 beschrieben. Mit diesem Mischklon 3MK1 (400 $\mu$g/ml G418; 10 $\mu$M Methotrexat (MTX); s. Tab. 2 der EP-A-0330 977) wurde durch limitierende Verdünnung eine Klonvereinzelung durchgeführt. Ein so erhaltener Einzelklon, 3MK1-3-B11 wurde in Kulturmedium (DME-Medium, das 10 % foetales Kälberserum (FKS), 400 $\mu$g/ml G418 und 10 $\mu$M MTX enthält) vermehrt (Zell-Linie 3MK1-3-B11).

b) Konstruktion eines Expressionsvektors für Gal $\alpha$-2,6-ST

Ein für Gal $\alpha$-2,6-ST kodierendes cDNA-Fragment (2,2 kb) aus dem Vektor pSial14L (Beispiel 1) wurde durch Spaltung mit den Restriktionsenzymen Hind III und XbaI erhalten und in den mit HindIII/XbaI geöffneten Vektor pAB3-1 (Zettlmeißl et al., Behring Inst.Mitt. Bd. 82 (1988), 127-143) kloniert. Das resultierende Plasmid, das die Gal $\alpha$-2,6-ST cDNA in der gewünschten Orientierung stromabwärts vom SV40 early Promotor trägt, wurde pABSial genannt (Fig. ).

c) Kotransfektion der Zell-Linie 3MK1-3-B11 mit einem Expressions-Plasmid für Gal $\alpha$ 2,6-ST (pAB Sial) und einem Expressionsplasmid für Glutaminsynthetase (pSVLGS1)

Die gemäß (a) erhaltene Zell-Linie 3MK1-B11 wurde 8 Passagen lang auf Wachstum in GMEM-Medium (Bebbington and Hentschel (1987), a.a.O.), das 10 % FKS, 400 $\mu$g/ml G418 und 10 $\mu$M MTX enthielt (GS-Medium) eingestellt. Die so erhaltenen Zellen exprimieren 11 ± 2 $\mu$g AT 40 III/$10^6$ Zellen/24 h und wurden mit Hilfe der in der EP-A-0330 971 beschriebenen Calcium phosphattechnik mit den Plasmiden pAB Sial (20 $\mu$g) und pSVLGS1 (5 $\mu$g; Bebbington und Hentschel (1987), a.a.O.) übertransfiziert. Übertransfizierte Zellen wurden in GS-Medium, das 15 $\mu$M Methionin-Sulfoximin (MSX) enthielt, selektioniert. Nach etwa 4 Wochen wurden 9 MSX resistente Einzelklone isoliert. Von drei dieser Klone (42-A3, 42-A11, 42-C1) wurde nach Standardverfahren RNA isoliert und im Northernblot mit einem radioaktiv markiertes Fragment der humanen Gal $\alpha$-2,6-ST cDNA als Sonde hybridisiert.

Alle 3 Klone enthielten im Gegensatz zum Ausgangsklon (3MK1-3-B11) eine Gal $\alpha$-2,6-ST spezifische mRNA. Das Gal $\alpha$-2,6-ST spezifische Signal in den Klonen 42-A3 und 42-C1 war in der Stärke vergleichbar mit dem Signal der AT III spezifischen mRNA, welches nach Übertransfektion mit den Vektoren pABSial/pSVLGS1 in allen 3 analysierten Klonen 42-A3, 42-A11 und 42-C1 im Vergleich zum Ausgangsklon 3MK1-3-B11 unbeeinflußt blieb .

Auch die Expressionsraten für Antithrombin III waren für alle 3 analysierten Gal $\alpha$-2,6-ST exprimierenden Zell-Linien im Vergleich zum Ausgangsklon identisch (11 ± 2 $\mu$g/$10^6$ Zellen/24 h).

Beispiel 3: Reinigung und Charakterisierung von menschlichem AT III aus einer BHK-Zell-Linie, die menschliches Gal $\alpha$-2,6-ST koexprimiert

a) Zellvermehrung und Reinigung von AT III

Die BHK Zell-Linien 3MK1-3-B11 (AT III) und 42-C1 (AT III + Gal $\alpha$-2,6-ST) wurden in GS-Medium in 1750 cm$^2$ Rollerflaschen bis zur Konfluenz gezüchtet. Nach Entfernen des GS-Mediums und einmaligem Waschen (4 Stunden) mit 100 ml serumfreien Iskove's Medium (Behringwerke AG, Marburg, FRG) wurden die Zellen viermal für je 48-72 h in 500 ml serumfreiem Iskove's Medium inkubiert. Für beide Zell-Linien wurden hierbei im Medium AT III-Konzentrationen größer als 12 mg/l erreicht. Die Reinigung des rekombinanten menschlichen AT III aus dem Kulturüberstand erfolgte nach einem Standardverfahren (Zettlmeißl et al. (1989), a.a.O.).

b) Sialinsäurebestimmung mittels HPAE-PAD

Die Bestimmung des Sialinsäuregehaltes der verschiedenen r-AT III-Proben erfolgte in an sich bekannter Weise (Hermentin und Seidat, a.a.O.) durch hydrolytische Freisetzung (0.1 N $H_2SO_4$, 1 h, 80 °C), Auftrennung ("high-pH anion-exchange chromatography", HPAE) und Bestimmung des Sialinsäuregehaltes durch "pulsed amperometric detection" (PAD). Bei der mit Gal $\alpha$-2,6-ST transfizierten Zell-Linie 42-C1 erwies sich der Sialinsäuregehalt gegenüber der nicht transfizierten Zell-Linie 3MK1-3-B11 um ca. 40 % erhöht.

c) Glycan-Differenzierung

Die mit Gal $\alpha$ 2,6-ST transfizierte Zell-Linie 42-C1 und die nicht transfizierte Zell-Linie 3MK1-3-B11 wurden hinsichtlich der Anknüpfung der Sialinsäure an terminale Galactosereste der auf AT III vorhandenen N-Glycane in an sich bekannter Weise mit Hilfe des "Glycan-Differentiation Kit" der Fa. Boehringer Mannheim (Ref.-Nr. 1142 372) untersucht. Bei diesem Test wird in einem sogen. "Dot Blot Assay" $\alpha$-2,3-verknüpfte Sialinsäure mit Hilfe des Digoxigenin tragenden Lectins MAA (Maackia amurensis agglutinin) und $\alpha$-2,6-verknüpfte Sialinsäure mit Hilfe des Digoxigenin tragenden Lectins SNA (Sambucus nigra agglutinin) erkannt und in einem "Sandwich-Assay" mit anti-Digoxigenin vom Schaf, welches mit dem Enzym alkalische Phosphatase konjugiert ist, über eine Farbreaktion nachgewiesen.

Als Kontrollen wurden folgende Standardglykoproteine des "Glycan Differentiation Kit" verwendet:

Fetuin: positiv für die Lectine MAA, SNA und DSA;

Asialofetuin: negativ für das Lectin SNA;

Transferrin : (Negativkontrolle)

Der Auftrag erfolgte jeweils in Verdünnungsreihen von 1.0, 0.5 und 0.2 ug Glycoprotein pro Vertiefung.

Bei aus der nicht-transfizierten Zell-Linie 3MK1-3-B11 isoliertem AT III wurde ausschließlich $\alpha$-2,3-

verknüpfte Sialinsäure nachgewiesen. Bei aus der mit Gal $\alpha$-2,6-ST transfizierten Zell-Linie 42-C1 isoliertem AT III wurde zusätzlich zur normalen $\alpha$-2,3-verknüpften Sialinsäure auch $\alpha$-2,6-verknüpfte Sialinsäure nachgewiesen (Tabelle 2).

Das Ergebnis belegt, daß die menschliche $\alpha$-2,6-Sialyltransferase nach Transfektion der umzurechnenden DNA-Sequenz in der 42-C1-Zell-Linie aktiv ist und zusätzlich zur normalen $\alpha$-2,3-Sialylierung eine $\alpha$-2,6-Sialylierung von menschlichem Antithrombin III ermöglicht.

Beispiel 4: In analoger Weise zu Beispielen 1-3 kann eine verbesserte Sialylierung von rekombinantem Erythropoietin aus verschiedenen eukaryontischen Zellinien erzielt werden. Besonders bevorzugt sind hierfür folgende Zellinien: BHK, C127, CHO

Tab. 1

```
          10                  30                  50
aattctGCCCGGCGTTAACAAAGGGAGCCGATACCGACCGGCGTGGGCGCGGAGCGGGCG

          70                  90                  110
GCCGCCACCGAGCGTGCTGAGCAACCGCAGCCTCCGCGGCCGAGAGTGCAGCGAGCAAGG

          130                 150                 170
GGAGAGCCAGTTGCGCAGAGCCCTGCAACCAGCAGTCCAGGGAGAAGTGGTGAATGTCAT

          190                 210                 230
GGAGCCCAGCTGAAATGGACTGGCCCCCTTGAGCCTGTCCCAAGCCCTGGTGCCAGGTGT

          250                 270                 290
CCATCCCCGTGCTGAGATGAGTTTTGATCATCCTGAGAAAAATGGGCCTTGGCCTGCAGA

          310                 330                 350
CCCAATAAACCTTCCCTCCCATGGATAATAGTGCTAATTCCTGAGGACCTGAAGGCCTGC

          370                 390                 410
CGCCCCTGGGGGATTAGCCAGAAGCAGGCTTGTTTTCCTGCTCAGAACAAAGTGACTTCC

          430                 450                 470
CTGAACACATCTTCATTATGATTCACACCAACCTGAAGAAAAAGTTCAGCTGCTGCGTCC
              M  I  H  T  N  L  K  K  K  F  S  C  C  V  L

          490                 510                 530
TGGTCTTTCTTCTGTTTGCAGTCATCTGTGTGTGGAAGGAAAAGAAGAAAGGGAGTTACT
 V  F  L  L  F  A  V  I  C  V  W  K  E  K  K  K  G  S  Y

          550                 570                 590
ATGATTCCTTTAAATTGCAAACCAAGGAATTCCAGGTGTTAAAGAGTCTGGGGAAATTGG
 D  S  F  K  L  Q  T  K  E  F  Q  V  L  K  S  L  G  K  L  A

          610                 630                 650
CCATGGGGTCTGATTCCCAGTCTGTATCCTCAAGCAGCACCCAGGACCCCCACAGGGGCC
 M  G  S  D  S  Q  S  V  S  S  S  T  Q  D  P  H  R  G  R

          670                 690                 710
GCCAGACCCTCGGCAGTCTCAGAGGCCTAGCCAAGGCCAAACCAGAGGCCTCCTTCCAGG
 Q  T  L  G  S  L  R  G  L  A  K  A  K  P  E  A  S  F  Q  V

          730                 750                 770
TGTGGAACAAGGACAGCTCTTCCAAAAACCTTATCCCTAGGCTGCAAAAGATCTGGAAGA
 W  N  K  D  S  S  S  K  N  L  I  P  R  L  Q  K  I  W  K  N

          790                 810                 830
ATTACCTAAGCATGAACAAGTACAAAGTGTCCTACAAGGGGCCAGGACCAGGCATCAAGT
 Y  L  S  M  N  K  Y  K  V  S  Y  K  G  P  G  P  G  I  K  F

          850                 870                 890
TCAGTGCAGAGGCCCTGCGCTGCCACCTCCGGGACCATGTGAATGTATCCATGGTAGAGG
 S  A  E  A  L  R  C  H  L  R  D  H  V  N  V  S  M  V  E  V

          910                 930                 950
TCACAGATTTTCCCTTCAATACCTCTGAATGGGAGGGTTATCTGCCCAAGGAGAGCATTA
 T  D  F  P  F  N  T  S  E  W  E  G  Y  L  P  K  E  S  I  R
```

7

```
          970                 990                1010
GGACCAAGGCTGGGCCTTGGGGCAGGTGTGCTGTTGTGTCGTCAGCGGGATCTCTGAAGT
    T  K  A  G  P  W  G  R  C  A  V  V  S  S  A  G  S  L  K  S

          1030                1050                1070
CCTCCCAACTAGGCAGAGAAATCGATGATCATGACGCAGTCCTGAGGTTTAATGGGGCAC
    S  Q  L  G  R  E  I  D  D  H  D  A  V  L  R  F  N  G  A  P

          1090                1110                1130
CCACAGCCAACTTCCAACAAGATGTGGGCACAAAAACTACCATTCGCCTGATGAACTCTC
    T  A  N  F  Q  Q  D  V  G  T  K  T · T  I  R  L  M  N  S  Q

          1150                1170                1190
AGTTGGTTACCACAGAGAAGCGCTTCCTCAAAGACAGTTTGTACAATGAAGGAATCCTAA
    L  V  T  T  E  K  R  F  L  K  D  S  L  Y  N  E  G  I  L  I

          1210                1230                1250
TTGTATGGGACCCATCTGTATACCACTCAGATATCCCAAAGTGGTACCAGAATCCGGATT
    V  W  D  P  S  V  Y  H  S  D  I  P  K  W  Y  Q  N  P  D  Y

          1270                1290                1310
ATAATTTCTTTAACAACTACAAGACTTATCGTAAGCTGCACCCCAATCAGCCCTTTTACA
    N  F  F  N  N  Y  K  T  Y  R  K  L  H  P  N  Q  P  F  Y  I

          1330                1350                1370
TCCTCAAGCCCCAGATGCCTTGGGAGCTATGGGACATTCTTCAAGAAATCTCCCCAGAAG
    L  K  P  Q  M  P  W  E  L  W  D  I  L  Q  E  I  S  P  E  E

          1390                1410                1430
AGATTCAGCCAAACCCCCCATCCTCTGGGATGCTTGGTATCATCATCATGATGACGCTGT
    I  Q  P  N  P  P  S  S  G  M  L  G  I  I  I  M  M  T  L  C

          1450                1470                1490
GTGACCAGGTGGATATTTATGAGTTCCTCCCATCCAAGCGCAAGACTGACGTGTGCTACT
    D  Q  V  D  I  Y  E  F  L  P  S  K  R  K  T  D  V  C  Y  Y

          1510                1530                1550
ACTACCAGAAGTTCTTCGATAGTGCCTGCACGATGGGTGCCTACCACCCGCTGCTCTATG
    Y  Q  K  F  F  D  S  A  C  T  M  G  A  Y  H  P  L  L  Y  E

          1570                1590                1610
AGAAGAATTTGGTGAAGCATCTCAACCAGGGCACAGATGAGGACATCTACCTGCTTGGAA
    K  N  L  V  K  H  L  N  Q  G  T  D  E  D  I  Y  L  L  G  K

          1630                1650                1670
AAGCCACACTGCCTGGCTTCCGGACCATTCACTGCTAAGCACAGGCTCCTCACTCTTCTC
    A  T  L  P  G  F  R  T  I  H  C

          1690                1710                1730
CATCAGGCATTAAATGAATGGTCTCTTGGCCACCCCAGCCTGGGAAGAACATTTTCCTGA

          1750                1770                1790
ACAATTCCAGCCTGCTCCTTTTACTCTAGGGGCCTCTGTCAGCAAGACCATGGGACTTCA

          1810                1830                1850
AGAGCCTGTGGTCAGGAAATCAGGTCCAGCCTTCCCTGTAGCCAGACAGTTTATGAGCCC

          1870                1890                1910
AGAGCCTCCTGCCACACACATGCACACATATCTAGCATTCTTTCCAAGACAGCATCCTCC
```

8

```
            1930                   1950                   1970
CCGCCTTCCACCTTGTAGATGCAAGGTCTATCTCTCCCATCAGGGCTGCCAAAGCTGGGC

            1990                   2010                   2030
TTTGTTTTTCCCAGCAGAATGATGCCATTCTCACAAACCAATGCTCTATATTGCTTGAAG

            2050                   2070                   2090
TCTGCATCTAAATATTGATTTCACGTTTTAAAGAAATTCTCTTAAATTACAATTGTGCCC

            2110                   2130                   2150
AATGCAGGGTGGCTCTGGGGGGCAAGTAGGTGGTACAGGGGATTGGAAACAATCGTCCGC

            2170            2188
GCCTCCAGAGAAAAGTTGCTCCCGAGag
```

**Patentansprüche**

1. Verfahren zur Sialylierung von Glykoproteinen, dadurch gekennzeichnet, daß man eine für eine

Tabelle 2 : **Bestimmung der Sialinsäureverknüpfung mittels des "Glycan Differentiation Kit" (Boehringer Mannheim)**

| Glycoprotein | Lectin MAA (NANA- 2,3-pos.) | | | Lectin SNA (NANA- 2,6-pos.) | | | Lectin DSA(ß-Gal und ß-GlcNAc-pos.) | | |
|---|---|---|---|---|---|---|---|---|---|
| AT III aus 42-C1 | + | (+) | - | +++ | +++ | ++ | + | (+) | - |
| AT III aus 3MK1-3-B11 | + | (+) | - | - | - | - | + | (+) | - |
| Fetuin (Positivkontrolle für MAA, SNA und DSA) | +++ | +++ | ++ | +++ | +++ | ++ | +++ | ++ | + |
| Transferrin (Negativkontrolle für MAA und DSA) | - | - | - | n.d. | n.d. | n.d. | - | - | - |
| Asialofetuin (Negativkontrolle für SNA) | n.d. | n.d. | n.d. | - | - | - | n.d. | n.d. | n.d. |
| | 1,0 | 0,5 | 0,2 | 1,0 | 0,5 | 0,2 | 1,0 | 0,5 | C,2 |
| | Auftrag (ug/well) | | | Auftrag (ug/well) | | | Auftrag (ug/well) | | |

10

EP 0 475 354 A2

Sialyltransferase kodierende DNA-Sequenz zusammen mit einer für das zu sialylierende Glykoprotein kodierenden DNA-Sequenz in einer eukaryontischen Zelle zur Expression bringt und das gebildete sialylierte Glykoprotein isoliert.

2. Verfahren nach Anspruch 1, dadurch gekennzeichnet, daß man die für eine Galaktosid $\alpha$-2,3-Sialyltransferase kodierende DNA-Sequenz verwendet.

3. Verfahren nach Anspruch 1, dadurch gekennzeichnet, daß man die für eine Galaktosid $\alpha$-2,6-Sialyltransferase kodierende DNA-Sequenz verwendet.

4. Verfahren nach Anspruch 1, dadurch gekennzeichnet, daß man die für N-Acetylgalactosaminosid $\alpha$-2,6-Sialyltransferase kodierende DNA-Sequenz verwendet.

5. Verfahren nach Anspruch 1, dadurch gekennzeichnet, daß man die für eine menschliche Galaktosid $\alpha$-2,3-Sialyltransferase kodierende DNA-Sequenz verwendet.

6. Verfahren nach Anspruch 1, dadurch gekennzeichnet, daß man die für eine menschliche Galaktosid $\alpha$-2,6-Sialyltransferase kodierende DNA-Sequenz verwendet.

7. Verfahren nach Anspruch 1, dadurch gekennzeichnet, daß man die für eine menschliche N-Acetylgalactosaminosid $\alpha$-2,6-Sialyltransferase kodierende DNA verwendet.

8. Verfahren nach Anspruch 1, dadurch gekennzeichnet, daß man zuerst die für die Sialyltransferase kodierende DNA-Sequenz zusammen mit einem Markergen und dann die für das zu sialylierende Glykoprotein kodierende DNA-Sequenz zusammen mit einem weiteren Markergen in die eukaryontische Zelle einbringt.

9. Verfahren nach Anspruch 1, dadurch gekennzeichnet, daß man zuerst die für das zu sialylierende Glykoprotein kodierende DNA-Sequenz zusammen mit einem Markergen und dann die für die Sialyltransferase kodierende DNA-Sequenz zusammen mit einem weiteren Markergen in die eukaryotische Zelle einbringt.

10. Verfahren nach Anspruch 1, dadurch gekennzeichnet, daß man die für die Sialyltransferase kodierende DNA-Sequenz und die für das zu sialylierende Glykoprotein kodierende DNA-Sequenz gleichzeitig zusammen mit einem Markergen in die eukaryotische Zelle einbringt.

11. Verfahren nach einem der Ansprüche 8 - 10 , dadurch gekennzeichnet, daß man als Markergene die kodierenden Sequenzen für Dihydrofolatreduktase, Glutaminsynthetase, Adenosindeaminase, Thymidinkinase, Puromycin N-Acetyltransferase, Aminoglykosid 3'Phosphotransferase verwendet.

12. Verfahren nach Anspruch 1, dadurch gekennzeichnet, daß man als eukaryotische Zellen Säugerzellen verwendet.

13. Verfahren nach Anspruch 1, dadurch gekennzeichnet, daß man als eukaryontische Zellen BHK-, C127-, CHO-, Maus-Myelom-, Vero- oder Hela-Zellen verwendet.

14. Verfahren zur Sialylierung von Glykoproteinen, dadurch gekennzeichnet, daß man eine für Sialyltransferase kodierende DNA Sequenz in eine Zelle einbringt, die natürlicherweise ein Glykoprotein bildet und das gebildete sialylierte Glykoprotein isoliert.

15. Verfahren nach Anspruch 14, dadurch gekennzeichnet, daß man als eukaryontische Zelle eine monoklonalen Antikörper produzierende Hybridomazelle verwendet.

16. Sialylierte Glykoproteine, erhältlich nach einem der Ansprüche 1 bis 15.

**Patentansprüche für folgenden Vertragsstaat: ES**

1. Verfahren zur Sialylierung von Glykoproteinen, dadurch gekennzeichnet, daß man eine für eine

11

Sialyltransferase kodierende DNA-Sequenz zusammen mit einer für das zu sialylierende Glykoprotein kodierenden DNA-Sequenz in einer eukaryontischen Zelle zur Expression bringt und das gebildete sialylierte Glykoprotein isoliert.

2. Verfahren nach Anspruch 1, dadurch gekennzeichnet, daß man die für eine Galaktosid $\alpha$-2,3-Sialyltransferase kodierende DNA-Sequenz verwendet.

3. Verfahren nach Anspruch 1, dadurch gekennzeichnet, daß man die für eine Galaktosid $\alpha$-2,6-Sialyltransferase kodierende DNA-Sequenz verwendet.

4. Verfahren nach Anspruch 1, dadurch gekennzeichnet, daß man die für N-Acetylgalactosaminosid $\alpha$-2,6-Sialyltransferase kodierende DNA-Sequenz verwendet.

5. Verfahren nach Anspruch 1, dadurch gekennzeichnet, daß man die für eine menschliche Galaktosid $\alpha$-2,3-Sialyltransferase kodierende DNA-Sequenz verwendet.

6. Verfahren nach Anspruch 1, dadurch gekennzeichnet, daß man die für eine menschliche Galaktosid $\alpha$-2,6-Sialyltransferase kodierende DNA-Sequenz verwendet.

7. Verfahren nach Anspruch 1, dadurch gekennzeichnet, daß man die für eine menschliche N-Acetylgalactosaminosid $\alpha$-2,6-Sialyltransferase kodierende DNA verwendet.

8. Verfahren nach Anspruch 1, dadurch gekennzeichnet, daß man zuerst die für die Sialyltransferase kodierende DNA-Sequenz zusammen mit einem Markergen und dann die für das zu sialylierende Glykoprotein kodierende DNA-Sequenz zusammen mit einem weiteren Markergen in die eukaryontische Zelle einbringt.

9. Verfahren nach Anspruch 1, dadurch gekennzeichnet, daß man zuerst die für das zu sialylierende Glykoprotein kodierende DNA-Sequenz zusammen mit einem Markergen und dann die für die Sialyltransferase kodierende DNA-Sequenz zusammen mit einem weiteren Markergen in die eukaryotische Zelle einbringt.

10. Verfahren nach Anspruch 1, dadurch gekennzeichnet, daß man die für die Sialyltransferase kodierende DNA-Sequenz und die für das zu sialylierende Glykoprotein kodierende DNA-Sequenz gleichzeitig zusammen mit einem Markergen in die eukaryotische Zelle einbringt.

11. Verfahren nach einem der Ansprüche 11-13, dadurch gekennzeichnet, daß man als Markergene die kodierenden Sequenzen für Dihydrofolatreduktase, Glutaminsynthetase, Adenosindeaminase, Thymidinkinase, Puromycin N-Acetyltransferase, Aminoglykosid 3'Phosphotransferase verwendet.

12. Verfahren nach Anspruch 1, dadurch gekennzeichnet, daß man als eukaryotische Zellen Säugerzellen verwendet.

13. Verfahren nach Anspruch 1, dadurch gekennzeichnet, daß man als eukaryontische Zellen BHK-, C127-, CHO-, Maus-Myelom-, Vero- oder Hela-Zellen verwendet.

14. Verfahren zur Sialylierung von Glykoproteinen, dadurch gekennzeichnet, daß man eine für Sialyltransferase kodierende DNA Sequenz in eine Zelle einbringt, die natürlicherweise ein Glykoprotein bildet und das gebildete sialylierte Glykoprotein isoliert.

15. Verfahren nach Anspruch 14, dadurch gekennzeichnet, daß man als eukaryontische Zelle eine monoklonalen Antikörper produzierende Hybridomazelle verwendet.